# EUROPEAN PATENT SPECIFICATION

(11) **EP 0 551 287 B1**
(45) Date of publication and mention of the grant of the patent: **29.01.1997**
(21) Application number: 91915398.1
(22) Date of filing: 10.09.1991
(51) Int. Cl.: A61M 5/50, A61M 5/32

(54) **SAFETY SYRINGE NEEDLE DEVICE WITH INTERCHANGEABLE AND RETRACTABLE NEEDLE PLATFORM**
SICHERHEITSSPRITZE MIT AUSTAUSCHBAREM UND EINZIEHBAREM NADELTRäGER
DISPOSITIF DE SECURITE POUR AIGUILLE DE SERINGUE A PORTE-AIGUILLE INTERCHANGEABLE ET RETRACTILE

(30) Priority: 03.10.1990 US 607127; 18.04.1991 US 687108
(43) Date of publication of application: 21.07.1993
(73) Proprietor: INVIRO MEDICAL DEVICES LTD., Bridgetown (BB)
(72) Inventor: NOVACEK, Laurel, A., Vancouver, British Columbia V6S 1G6 (CA); SHARP, Fraser, R., Vancouver, British Columbia V6I 1C5 (CA); MCLEAN, Donald, A., Vancouver, British Columbia V6R 1V3 (CA)
(74) Representative: Raynor, John
(86) International application number: PCT/CA91/00321
(87) International publication number: WO 92/05821

(56) References cited:
- EP-A- 0 327 061
- EP-A- 0 347 742
- WO-A-89/08468
- WO-A-89/12475
- WO-A-90/11099
- US-A- 4 747 830
- US-A- 4 861 338
- US-A- 4 955 870

## Description

This invention relates to a novel syringe, and in particular to a syringe with a needle for medical and industrial application, more particularly to a syringe which after being used by a person to inject medication or fluid into a patient, or withdraw fluids from a patient or after sampling or exposure to toxic materials, or the like, can be transformed to withdraw the needle into the syringe barrel for disposal purposes, thereby eliminating needle stick injuries.

This invention provides a syringe comprising:
a hollow elongate barrel;
an element carried by the barrel for mounting a needle at a distal end thereof and providing for fluid communication with the interior of the hollow barrel ;
and a plunger in the barrel moveable between advanced and withdrawn positions;
characterised in that the syringe also includes
a seal between the plunger and the barrel at the proximal end of the barrel for sealing the interior of the barrel at its proximal end when the plunger is in its advanced position;
and means for sealing the barrel at its distal end,
the said seals being such as to permit sterile conditions to be maintained in the syringe prior to use.

The syringe may be packaged and sterilized in bulk and not necessarily in individual packages. However, this does not prevent tampering or inadvertent disruption of seals and contamination. It is desirable therefore, that the syringe embody tamperproof features. To that end, in one embodiment hereof, tamperproofing can be accomplished by applying a strip of tape having adhesive along one face along the side of the syringe covering both the junctures of the needle guard and body of the syringe and the proximal end of the barrel and plunger. Thus, if the tape has been broken or is twisted or otherwise removed from the syringe, it would be evidence that the seals at the opposite ends of the syringe have possibly been disrupted. Similarly, in another embodiment hereof, a tight shrink-wrap of plastic material may be provided about these junctures. Again, removal of the shrink-wrapped plastic material or a twisting or severing thereof would indicate a possible disruption of or tampering with the seals. In a further form of the invention, the syringe may be dipped into a plastic material in liquid form whereby a thin-film coating is applied to and solidified about the entire outer surface of the syringe, including any needle guard and projecting portion of the plunger. Consequently, sterility may be maintained, provided the thin-film plastic coating is not broken, while at the same time, disruption of or tampering with the seals at the aforementioned junctures is readily evident.

A number of preferred embodiments of the present invention will now be described with reference to the accompanying drawings in which:-
Figure 1 is a perspective view partially in phantom of a syringe, with an attached needle guard according to the present invention;
Figure 2 is a longitudinal cross-sectional view thereof;
Figure 3 is a perspective view partially in section of the adapter and the distal end of the plunger illustrating the mating relationship of these elements;
Figure 6 is a different side elevational view of the syringe in partial sectioned view and with the needle guard removed;
Figure 7 illustrates a manner in which the plunger may be rotated with respect to the barrel in order to disconnect the needle assembly from the barrel for withdrawal of the assembly into the barrel;
Figure 8 is a cross-sectional view similar to Figure 2 but with the needle assembly unfastened from the distal end of the barrel and with the assembly withdrawn into the barrel;
Figure 9 is a cross-sectional view similar to that illustrated in Figure 8 but with the hollow cylindrical portion of the plunger broken away at the break point;
Figure 10 is a cross-elevational view partially in section with the hollow cylindrical plunger portion fastened to the distal end of the barrel after the cylindrical plunger portion has been broken away at the break point;
Figure 17 is an enlarged partial sectional view of ridges and an intervening groove on the inside surface of the barrel at the distal end and a corresponding annular ridge on the exterior surface of the hollow portion of the plunger which cooperates with the annular barrel ridges for locking and sealing purposes;
Figure 18 is a side elevational view partially in cross-section of a still further embodiment of the invention;
Figure 19 is a side elevational view partially in section of the embodiment of Figure 10 illustrating the cylindrical plunger portion disconnected from the needle end portion of the plunger;
Figure 20 illustrates a partial end view of the plunger taken along line 20-20 in Figure 11;
Figure 21 is a perspective view of one form or a tamperproof syringe constructed in accordance with the present invention;
Figure 22 is an enlarged fragmentary cross-sectional view with parts broken out illustrating another form of a tamperproof syringe;
Figure 23 is an enlarged side elevational view with parts broken out and illustrates the opposite ends of a syringe having a tamperproof coating according to the present invention.
(There are no Figures 4, 5, 11, 12, 13, 14, 15, 16, 24, 25 or 26).

The syringe of the invention includes a seal between the plunger and the barrel at the proximal end of the barrel for sealing the interior of the barrel at its proximal end when the plunger is in its advanced position. In particular, the invention concerns a syringe which after being used by a health care worker or hazardous industries worker, or the like, the inject medication or fluid into a patient, or withdraw fluid from a patient, or in sampling toxic material, for example, in an industrial process, can be transformed by the worker to withdraw the needle into the barrel of the syringe for disposal purposes, thereby eliminating potentially harmful needle stick injuries among such workers. in industrial applications, the storage of a contaminated needle is similarly effected within the barrel to prevent further contamination of the environment or process.

With any of the various embodiments of the basic syringe design referred to below, the needle is retracted by the user into the interior of the body of the syringe immediately after it is withdrawn from the patient's body tissue, or after exposure to hazardous situations. Thus, the needle is not exposed for accidental contact at any time after the needle has contacted the potentially hazardous patient's body fluids, or other hazardous materials. This retraction feature eliminates the possibility of potentially dangerous needle stick injuries occurring with contaminated needles.

Most disposable syringes can be used with a variety of interchangeable needles with different diameter and length connected to the needle barrel by a Liner connector, which may be of two types. One is a simple conical device which accepts the needle base, known as a Luer tip. To detach the needle, it is simply pulled off. The other connector type is known as a Luer lock. The Luer lock has a simply screw thread locking mechanism that permits the base of the needle to be screwed onto the syringe so that it cannot be pulled off without unscrewing. In this disclosure, the universal coupling mechanism connecting the needle to the syringe will be referred to as a Luer lock version of the Luer connector unless otherwise indicated while the claims cover both the plain Luer tip and the Luer lock mechanisms.

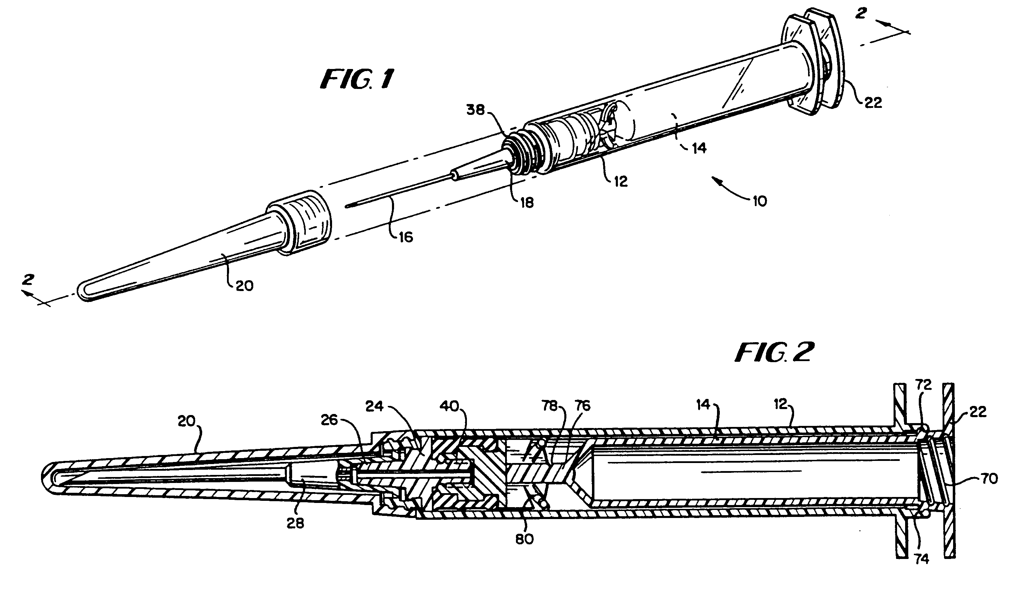

Referring now to the drawings, particularly to Figures 1-3, there is illustrated a syringe generally designated (10), comprised of a barrel (12), a plunger (14), a needle (16) attached to a Luer lock (18), and a needle guard (20). A finger press (22) is provided at the remove end of plunger (14). Also, at the end of the barrel remote from the plunger and thereof, there is provided an adapter (24) which includes a tapered end (26) for receiving a Luer lock (28).

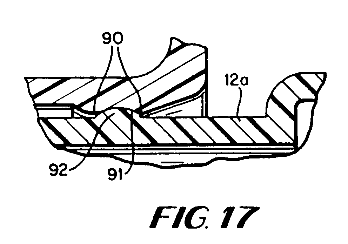

Referring to Figure 17, barrel (12a) includes elements (90) which, as illustrated in greater detail in Figure 17, include two annular ridges and an intervening groove (91) on the inside surface at the finger press end of the barrel. As will be seen in Figure 17, the groove (91) of the barrel is designed to cooperate with annular ridge (92) included in plunger. As will be recognised, these parts can be reversed. That is to say, groove (91) can be included on the plunger and ridge (92) can be included on the barrel. These elements cooperate when engaged to form an effective seal at one end of the barrel so as to prevent ingress or egress of materials to the central portion of the syringe body until it is used. This seal along with the use of a needle guard of the nature illustrated with the embodiment of Figure 2, which may be threaded to the barrel, will completely seal both ends of the syringe body. Such sealing will allow the syringe to be sterilized and the bulk packaged rather than individually packaged, but nevertheless, retain internal sterility.

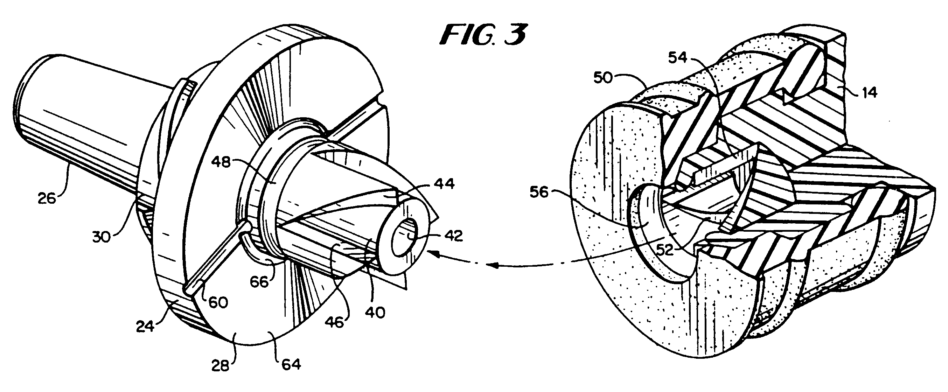

Referring particularly to Figure 3, adapter (24) is generally cylindrical, having an annular section (28) for butting against a shoulder (30) at the end of barrel 12. A reduced diameter section has an external thread, for example, a lefthanded thread 32, for engaging with mating threads 34 on the reduced diameter end 36 of the barrel. For reasons which will become apparent, the reduced diameter end 36 is also externally threaded at 38.

Projecting axially from the opposite end of adapter 24 is a protrusion 40 which, when the adapter 24 is secured in the barrel end, projects into the interior chamber of barrel 12. Adapter 24 includes an axially extending tapered channel or passageway 42 for communicating between the interior of the barrel and the needle. Engagement structure extends about the external surface of protrusion 40 for engaging with a corresponding engagement structure on the plunger whereby the adapter may be removed from the barrel end and withdrawn, together with the needle, into the interior of the barrel. The engagement structure includes on the external surface of protrusion 40 non-jamming, fast-acting spiral threads or ramps 44, together with axially extending end stops 46. Below ramps 44 is an annular groove 48 which cooperates with the connecting structure on the end of the plunger.

Referring to Figure 3, the end of the plunger 14 includes a bung 50 formed of resilient material and which surrounds a mating engagement structure on the plunger end for engaging with the structure about protrusion 40. The engagement structure on the plunger includes ramps and end faces 52 and 54, respectively, complementary to the ramps 44 and end faces 46 on protrusion 40. At the distal end of the plunger, there is an inwardly projecting annular ring 56 for engaging in the annular groove 48. Thus, when the plunger is advanced within the barrel to engage protrusion 40, the complementary ramps 44 and 52 engage one another. Due to the slopes of the ramps 44 and 52, the plunger is rotated in one direction relative to the adapter upon axial movement of the plunger toward the adapter to engage the end faces 46 and 54 with one another. Further, rotation of the plunger in one direction causes a unidirectional torque to be applied to the adapter, enabling the adapter to be unthreaded from the barrel end. Plunger rotation in the opposite direction causes the end stops 54 and 46 to separate and the plunger to ratchet relative to the adapter.

In operation, and after injection, the plunger is advanced axially and guided by the engaging complementary ramps 44 and 52 to engage end stops 46 and 54. Further axial advancement causes full insertion of the plunger into the barrel to engage ridge 56 in groove 48. Further rotation of the plunger in one direction unthreads adapter 24 from barrel end threads 34 while maintaining the adapter attached to the plunger end by the engagement of ridge 56 and groove 48. Ridge 56 may be partially or wholly annular as desired.

To ensure that air from the interior of the barrel may be ejected therefrom prior to use of the syringe, there is provided, with reference to Figure 3, a pair of vent passages 60 extending radially from and in communication with the central axial passageway 42 through vents 61. These passages preferably extend in grooved portions formed in the face of the flange facing the proximal end of the barrel. Additionally, the face 64 of the adapter facing the proximal end is tapered radially inwardly in a direction toward the needle end of the barrel, for example, on the order of about 3°. Further, an annular trough or groove 66 is preferably formed in the tapered flange face about the base of protrusion 40. Groove 66 lies in communication with the radial grooves 60 and vents 61 extending into the central passageway 42. One or more additional annular grooves may also be provided, including about the outer margin of the tapered flange base or at intermediate radial positions. By grooving the face of the adapter and tapering that face, grooves 60 and 66 will assume the most superior position within the interior of the barrel when the syringe is oriented vertically with the needle uppermost. Consequently, when the plunger is advanced toward the needle end to vent air from the interior of the barrel, as customary, any air trapped within the annular space between the distal end of the barrel and protrusion 40 will vent through grooves 60, 66 and vents 61 into central passageway 42. Hence, the interior of the barrel may be purged of any air prior to injection.

Referring back now to Figures 1 and 2, it will be appreciated that needle guard 20 covers the needle and is affixed to the barrel by barrel threads 38 and complementary threads on the guard. Plunger 14 also includes internal threads 70 adjacent finger press 22, together with an annular ridge 72 for providing sealing engagement with a groove 74 formed internally along the distal end of the barrel.

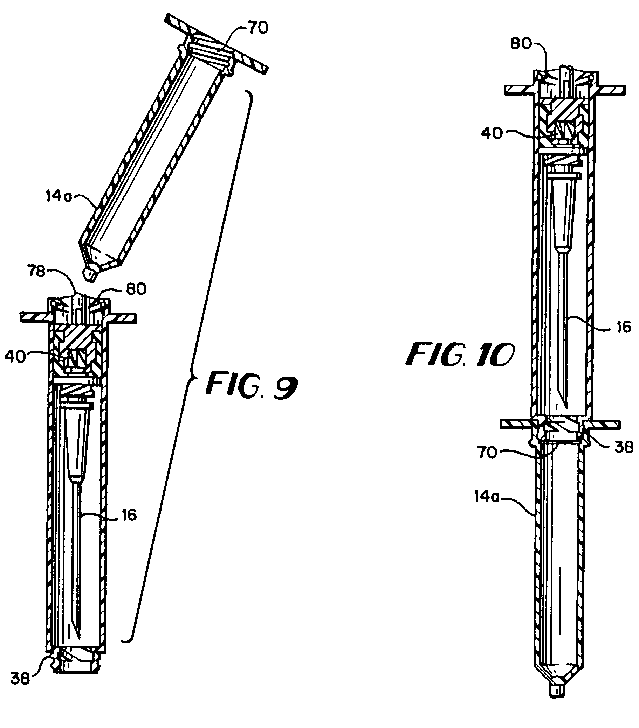

Once the syringe has been sterilized and assembled, as illustrated in Figure 2, with the needle guard in place forming a sealing engagement with the threaded barrel, as well as with the plunger in the position shown, forming a sealing engagement between elements 72 and 74, the interior of the structure is thus sealed at both ends, maintaining sterile conditions. Other forms of seals may be used. For example, friction fits or annular ridges and complementary grooves may be used. Note that the threads 38 accommodate both the threaded connection with needle guard 20 as well as the threaded connection with threads 70 of the hollow plunger portion once the plunger is broken away and connected to the end of the barrel, as illustrated in Figures 9 and 10.

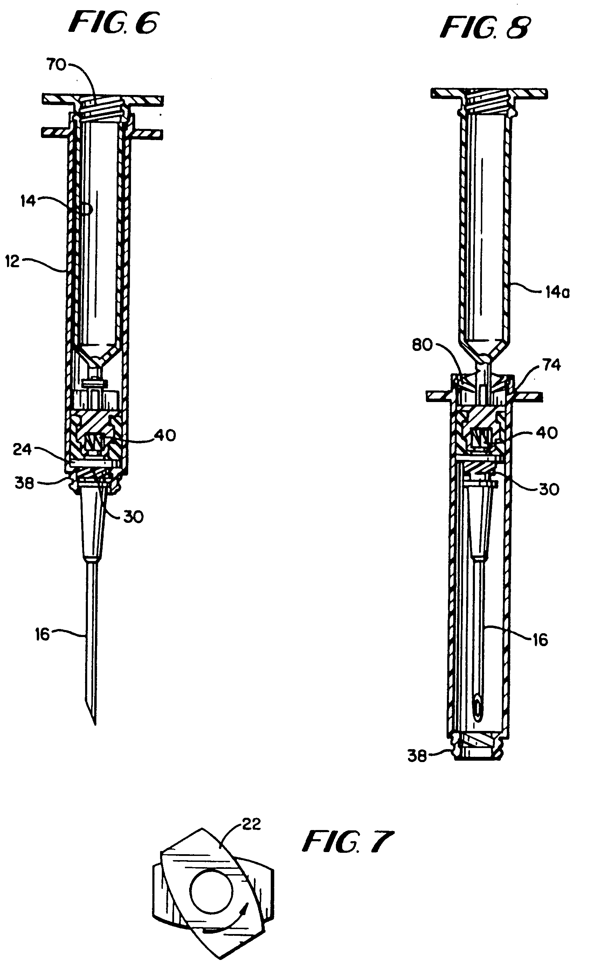

After use, and with the adapter and plunger connected, as illustrated in Figure 2, the plunger may be rotated in the manner shown in Figure 7 to disconnect the adapter from the interior threads at the distal end of the barrel as previously described. An important feature of this engagement mechanism is that once the adapter has been disconnected at its threaded connection with the barrel, it, along with the remainder of the needle assembly, remain attached to the plunger portion through the snap connection formed by the ridge 56 and groove 48. Thereafter, the plunger and attached needle assembly may be withdrawn into the barrel.

Retraction of the contaminated needle into the barrel after use prevents or substantially reduces the possibility of inadvertent needle stick injuries. Furthermore, it will be appreciated that once adapter 24 has been disconnected from the distal end of barrel 12, in the absence of extraordinary measures or the use of a special tool, the needle assembly and the adapter cannot be reassembled to the barrel and the syringe reused. Thus, attempts to reattach the adapter to the barrel for subsequent use, illicit or otherwise, is effectively prevented since the ramps (spiral grooves) and end stop faces of the adapter-plunger connection are shaped to prevent transfer of torque in a direction to attain reattachment of the adapter to the barrel by rotating the plunger in the direction opposite that illustrated in Figure 7.

In use, the exemplary embodiment of syringes as illustrated, are bulk packaged, sterilized, assembled structures of the nature generally illustrated in Figure 2. Initially, needle guard 20 is removed, the plunger and attached bung are withdrawn, and the proper dosage of fluid within the syringe body is adjusted using calibration markings (not shown) on the barrel in combination with the straight radial forward edge of the bung 50 to obtain a rapid and highly accurate measured reading of the contents.

After use and when the plunger and needle assembly including adapter 24 are withdrawn to the position illustrated in Figure 8, the ends of the resilient arms 80 register in the annular groove 74 so as to retain the needle assembly and lower portion of the plunger in the position illustrated. Thereafter, cylindrical plunger portion 14a may be broken away at the plunger break point 78 in the manner illustrated in Figure 9. The narrowed portion of the plunger 76 adjacent break point 78 is circular in cross-section in Figure 11 but may take other shapes such as oval to enhance the ease with which portion 14a may be broken away. After portion 14a has been broken away, it may be attached to the threaded distal end of the barrel as illustrated in Figure 10.

Thus, the needle assembly is locked in the barrel and by locking arm 80. Moreover, the Dung serves to seal one end of the barrel. Also, portion 14a of the plunger may be attached to the threaded end of barrel 12 by the illustrated threaded connection or by other well known snap-fit or friction-fit connections, for example. As illustrated in Figure 10, not only is the needle withdrawn to a position eliminating inadvertent needle sticks, but the barrel is sealed at both ends thus effectively preventing the escape of any toxic or contagious contents.

It will be appreciated that because plunger element 14a is hollow and is made sufficiently long as to encapsulate the needle assembly in the position illustrated in Figure 2, for example, should the adapter 24 malfunction and fail to separate from the barrel, the exposed needle assembly may nevertheless be covered by either needle guard 20 or by plunger portion 14a. In this regard because the needle guard, as well as the hollow plunger portion, have been made of approximately the same diameter as the threaded end of the barrel, both the needle guard and the plunger portion 14a may more safely be installed on the exposed needle. Accordingly, it will be seen that the present design includes redundancy features whereby even if the needle assembly fails to disengage and retract after use, it may nevertheless be covered in at least two other ways as discussed above, thus incorporating additional safety features.

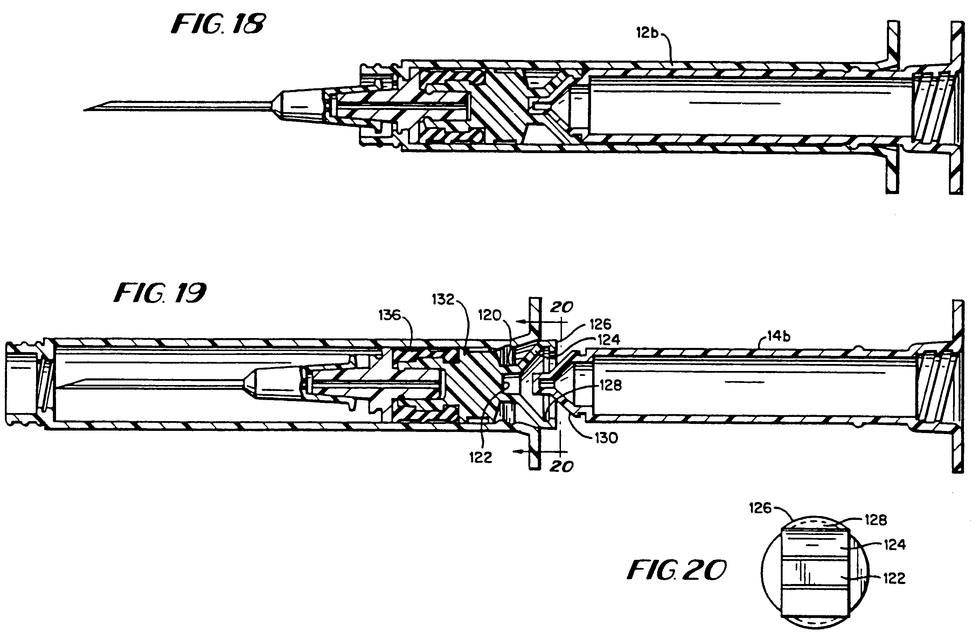

A further modification is illustrated in Figures 18 through 20. This embodiment is similar in most respects except that the narrowed intermediate portion of the plunger includes as a substitute for a break point a connection whereby the hollow cylindrical portion of the plunger may be unlatched or disconnected from the needle end portion and subsequently re-attached thereto. Such construction allows the hollow finger press portion of the plunger 14b to be used as a needle guard both before and after use, as well as a plunger portion during use.

In Figure 18, the needle end plunger portions are substantially the same except that the narrowed intermediate portion with break point 78 has been replaced with a rectilinear locking mechanism 120 having a rectangular shaped socket 122 with two resilient wings or side arms 124 that are slightly flared in the outward or radial direction. The upper ends of these two arms include lips 126 which are arcuate and have internal grooves 128 which are also arcuate.

The plunger portion 14b is a hollow cylindrical cylinder in cross section. However, the needle end portion thereof has been modified as illustrated in Figures 18 and 19 to include a rectilinear portion 130 which is complementary to the rectilinear portion 120. Accordingly, when these portions are joined, both portions 14b and 132 will rotate as a unit. Moreover, since the adapter/plunger engagement structure in this embodiment is, for example, unidirectional rotation of the adapter will be obtained in the previously described manner when the plunger is rotated.

Note that the same type of sealing at the finger press end of the syringe comprising complementary ridge arrangements is included in this embodiment. The finger press end of barrel 12b, however, has been tapered to enable arms 124 to flare outwardly and unlock or disengage from the complementary grooves included in plunger portion 14b.

In operation the sterilized syringe is supplied with cylindrical portion 14b connected over the needle and in sealing engagement with the distal end of the barrel 12b. In this regard, although the sealing engagement is illustrated as a threaded connection, other connection means as previously described may be used. As supplied, the syringe would also include the adapter and needle assembly connected to the distal end of the barrel but with the plunger portions 132 and 120 along with bung 136 withdrawn into the barrel and in sealing engagement with the finger press end of the barrel in the same manner noted with regard to the embodiment of Figure 13. Additionally, arms 124 would be positioned as illustrated in Figure 19. (Note, however, that the arms 124 in their unbiased condition may be smaller in diameter than the diameter of the barrel, thus enabling similar attachment of the plunger when the arms 124 lie within the barrel). Accordingly, as supplied, both ends of the syringe would be sealed, thus maintaining sterility as to the enclosed portions of the syringe body. Additionally, once the syringe is used the needle end portion of the plunger along with the adapter and needle assembly may be withdrawn and plunger portion 14b disconnected, as illustrated in Figure 19. Thereafter, the disconnected plunger portion 14b may be reconnected as before to the distal needle end portion of the barrel so that both ends of the syringe are again sealed whereby toxic or contagious material within the syringe body cannot escape.

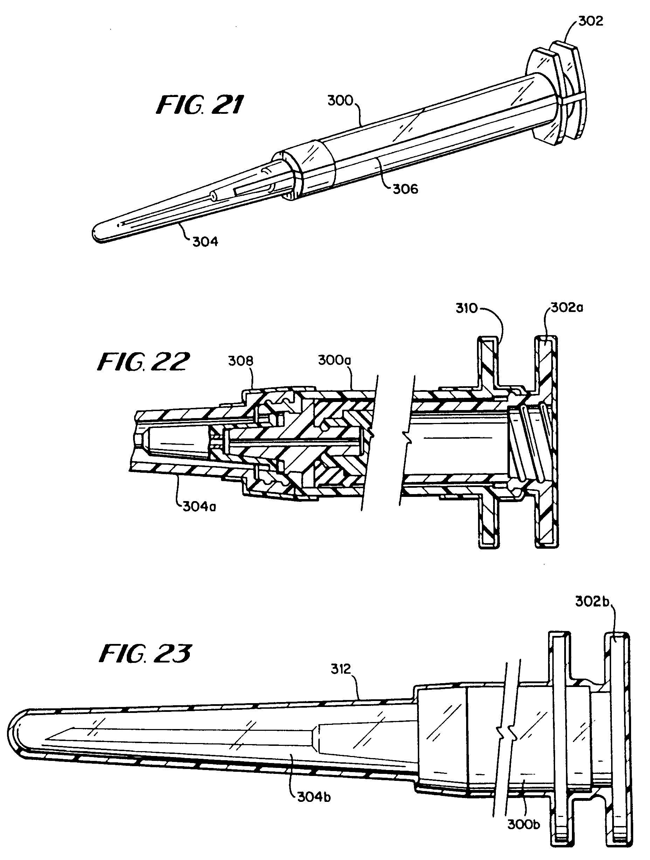

Referring now to the embodiments of the invention illustrated in Figures 21-23, three forms of syringes having tamperproof features are disclosed, respectively. In Figure 21, there is illustrated a syringe having a barrel 300, a finger press 302 for the plunger, and a needle guard 304. The needle guard is, of course, screw-threaded or otherwise secured to the end of the barrel 300, for example, as illustrated in Figures 21 and 22. In this form of the invention, a strip 306 of paper or other material, such as plastic, having adhesive along one side thereof is applied along a side of the syringe. Particularly, the strip is applied along the side of the barrel 300 and onto the end of the needle guard 304 at its juncture with the barrel 300. The opposite end of the strip 306 is applied to the finger press 302 and preferably extends across the top of the finger press and partially down the opposite side to at least the cylindrical barrel portion. In this manner, the juncture between the finger press 302 and barrel is spanned by at least a portion of the strip 306. As a result, it will be appreciated that any inadvertent or attempted removal of the needle guard from the barrel or rotational or axial movement of the finger press 302 relative to the barrel will cause the strip 306 to tear or become twisted whereby the disruption of the seal at these junctures and possible tampering with the syringe will be indicated.

In Figure 22, another form of tamperproof syringe is disclosed. In the embodiment illustrated in Figure 22, the barrel, finger press and needle guard are indicated as in Figure 21 with the suffix "a" applied thereto. In this form, thin-film plastic material 308 and 310, respectively, is applied, for example, by heat-shrinking, about each of the junctures between the needle guard 304a and barrel 300a on the one hand and the barrel 300a and finger press and plunger portion 302a, on the other hand. Once again, any rotary movement or other movement of the needle guard 304a relative to barrel 300a or rotary or axial movement of the finger press 302a relative to barrel 300a will be detected by the severing of the shrink-wrapped plastic material or its wrinkling whereby disruption of the seals at the opposite ends of the syringe or tampering with the syringe will be indicated. It will also be appreciated that the shrink-wrap of plastic material assists in maintaining the sterility of the syringe and that suitable tear strips may be provided along each of the shrink-wrapped portions to facilitate their removal and use of the syringe.

In Figure 23, the like elements of the syringe are illustrated by like reference numerals as in Figures 31 and 22, followed by the suffix "b". In this form, the syringe is dipped into a melted plastic material whereby a thin-film coating of plastic 312 is applied about and completely envelops the syringe with the needle guard attached and plunger located in its axially innermost position. For example, the plastic coat 312 may comprise a clear chlorinated polyvinyl chloride coating (CPCV). The ingredients are vinyl chloride-vinyl acetate resin silicon dioxide (amorphus) with a methol isobutyl ketone base. This is an air dry coating. The drying can be accelerated by raising the temperature to 100°F. The melting temperature of the plastic material is, of course, less than the melting temperature of the plastic forming the syringe, and it will be appreciated that a clear plastic material should be used such that the gradations and other information on the syringe may be visualized through the thin-film plastic coating. Thus, any efforts to remove the needle guard 304b or displace the plunger will be immediately detectable by the break in the thin-film 12 of plastic material, hence indicating disruption of or tampering with the seals adjacent the opposite ends of the syringe. The coating material is, of course, sufficiently thin to enable the needle guard to be readily removed from the barrel and the plunger to be readily rotated to break the seal, hence permitting axial movement of the plunger relative to the barrel. Therefore, the plastic coating can be left on the syringe barrel during use, eliminating the need to physically strip it from the barrel and dispose of it separately.

As will be apparent to those skilled in the art in the light of the foregoing disclosure, many alterations and modifications are possible in the practice of this invention without departing from the scope thereof. Accordingly, the scope of the invention is to be construed in accordance with the substance defined by the following claims.

## Claims

1. A syringe comprising:
a hollow elongate barrel (12);
an element (18) carried by the barrel for mounting a needle at a distal end thereof and providing for fluid communication with the interior of the hollow barrel (18) ;
and a plunger (14) in the barrel moveable between advanced and withdrawn positions;
characterised in that the syringe also includes
a seal (72,74) between the plunger and the barrel at the proximal end of the barrel (12) for sealing the interior of the barrel (12) at its proximal end when the plunger (14) is in its advanced position;
and means (20) for sealing the barrel at its distal end,
the said seals (72,74,20) being such as to permit sterile conditions to be maintained in the syringe prior to use.

2. A syringe according to Claim 1, wherein the said element (18) is secured to the distal end (26) of the barrel (12) and has the needle projecting therefrom, and wherein the said sealing means at the distal end of the barrel comprises a needle guard (20) encompassing the needle and sealingly secured to said barrel.

3. A syringe according to Claim 1 or Claim 2, wherein the said sealing means (20) at the distal end of the barrel comprises a cap screw-threaded onto the distal end of the barrel.

4. A syringe as claimed in Claim 3, including means (308,310) connected to the barrel and the cap to indicate attempted movement of the plunger and the cap relative to one another.

5. A syringe as claimed in Claim 2, wherein means are provided connected to the barrel and the needle guard to indicate attempted movement of the needle guard and the barrel relative to one another.

6. A syringe as claimed in any one of the preceding Claims, including means (310) connecting the barrel and the plunger in the advanced position of the plunger to indicate attempted movement of the plunger and the barrel relative to one another.

7. A syringe as claimed in any one of the preceding Claims, wherein the seal at the proximal end includes a rib (72) on one of the said barrel and the said plunger and means (74) cooperating with the said rib for forming the said seal on the other of the said barrel and the said plunger.

8. A syringe as claimed in any one of the preceding Claims, wherein the proximal end of the plunger forms a hollow cylinder and includes connection structure sized and designed to cooperate with complementary connection structure included at the distal end of the barrel, and wherein the plunger further includes structure intermediate its proximal and distal ends for detachably connecting the said proximal and distal ends, whereby when the plunger is withdrawn from the distal end of the barrel, the proximal end of the plunger can be detached from the plunger and connected to the distal end of the barrel.

9. A syringe according to any one of the preceding Claims including an adapter (24) carried by the barrel (12) adjacent to the distal end thereof and removable therefrom in response to rotation relative to the barrel (12) the adapter carrying a needle, and wherein the syringe includes engagement structure for the adapter disposed at the distal end of the plunger (14) and engageable with a mating connection engagement structure on the adapter (24), said structures having respective drive and connective engagement surfaces (48,46,56,54), said drive surfaces being engageable with one another in response to axial movement of said plunger (14) toward the distal end of said barrel (12) and jointly movable upon engagement to enable rotation of the adapter (24) relative to the barrel (12) in response to relative rotation of the plunger (14) and barrel (12) to cause the adapter (24) to part from the distal end of the barrel (12) and wherein the connective surfaces (48, 56) are engageable with one another to connect the plunger and adapter one with the other and enable said adapter (24), when parted from the end of the barrel (12) in response to joint rotation of said adapter (24) and said plunger (14) relative to said barrel (12) to be withdrawn with the needle into the interior of the barrel in response to joint axial movement of said plunger (14) and said adapter (24) in a direction away from the distal end of barrel (12).

10. A syringe according to Claim 9 wherein the adapter engagement structure at the distal end of the plunger (14) and the mating connection engagement structure on the adapter (24) include respective surfaces cooperable to cause relative rotation of the plunger (14) and adapter (24) in response to axial movement of the plunger (14) toward the distal end of the barrel.

11. A syringe according to Claim 9 or Claim 10 wherein the adapter engagement structure at the distal end of the plunger (14) and the mating connection engagement structure on the adapter (24) include respective alignment surfaces (44, 52) different from the drive surfaces (46, 54) and cooperable to cause relative rotation of the plunger and adapter in response to axial movement of the plunger toward the distal end of the barrel.

## Patentansprüche

1. Spritze mit:
einem hohlen länglichen Gehäuse (12);
einem von dem Gehäuse getragenen Element (18) zum Montieren einer Nadel an dessen distalem Ende und zum Bereitstellen einer Flüssigkeitsverbindung mit dem Inneren des hohlen Gehäuses (18);
und einem Kolben (14), der in dem Gehäuse zwischen vorgeschobenen und zurückgezogenen Positionen bewegbar ist;
dadurch gekennzeichnet, daß die Spritze ferner aufweist eine Dichtung (72, 74) zwischen dem Kolben und dem Gehäuse am proximalen Ende des Gehäuses (12) zum Abdichten des Inneren des Gehäuses (12) an seinem proximalen Ende, wenn der Kolben (14) in seiner vorgeschobenen Position ist;
und eine Einrichtung (20) zum Abdichten des Gehäuses an seinem distalen Ende,
wobei die Dichtungen (72, 74, 20) derart sind, daß sie das Aufrechterhalten steriler Bedingungen in der Spritze vor der Verwendung zulassen.

2. Spritze nach Anspruch 1, wobei das Element (18) am distalen Ende (26) des Gehäuses (12) befestigt ist und die Nadel davon vorspringt, und wobei die Dichtungseinrichtung am distalen Ende des Gehäuses einen Nadelschutz (20) aufweist, der die Nadel umgibt und dichtend an dem Gehäuse befestigt ist.

3. Spritze nach Anspruch 1 oder Anspruch 2, wobei die Dichtungseinrichtung (20) am distalen Ende des Gehäuses eine Kappe aufweist, die mittels Schraubgewinde auf das distale Ende des Gehäuses aufgeschraubt ist.

4. Spritze nach Anspruch 3, die eine mit dem Gehäuse und der Kappe verbundene Einrichtung (308, 310) aufweist, um eine versuchte Bewegung des Kolbens und der Kappe relativ zueinander anzuzeigen.

5. Spritze nach Anspruch 2, wobei eine mit dem Gehäuse und dem Nadelschutz verbundene Einrichtung vorgesehen ist, um eine versuchte Bewegung des Nadelschutzes und des Gehäuses relativ zueinander anzuzeigen.

6. Spritze nach einem der vorhergehenden Ansprüche, die eine das Gehäuse und den Kolben in der vorgeschobenen Position des Kolbens verbindende Einrichtung (310) aufweist, um eine versuchte Bewegung des Kolbens und des Gehäuses relativ zueinander anzuzeigen.

7. Spritze nach einem der vorhergehenden Ansprüche, wobei die Dichtung am proximalen Ende eine Rippe (72) entweder an dem Gehäuse oder an dem Kolben und eine Einrichtung (74) aufweist, die mit der Rippe zum Bilden der Dichtung an der jeweils anderen Komponente, dem Gehäuse oder dem Kolben, zusammenwirkt.

8. Spritze nach einem der vorhergehenden Ansprüche, wobei das proximale Ende des Kolbens einen hohlen Zylinder bildet und eine Verbindungsstruktur aufweist, die dazu dimensioniert und konstruiert ist, um mit einer an dem distalen Ende des Gehäuses vorgesehenen komplementären Verbindungsstruktur zusammenzuwirken, und wobei der Kolben ferner zwischen seinem proximalen und seinem distalen Ende eine Struktur zum lösbaren Verbinden des proximalen und des distalen Endes aufweist, wodurch das proximale Ende des Kolbens von dem Kolben abgenommen und mit dem distalen Ende des Gehäuses verbunden werden kann, wenn der Kolben von dem distalen Ende des Gehäuses zurückgezogen ist.

9. Spritze nach einem der vorhergehenden Ansprüche, mit einem Adapter (24), der von dem Gehäuse (12) nahe an dessen distalem Ende getragen wird und in Antwort auf eine Drehbewegung relativ zu dem Gehäuse (12) davon entfernbar ist, wobei der Adapter eine Nadel trägt, und wobei die Spritze eine Eingriffsstruktur für den Adapter aufweist, die am distalen Ende des Kolbens (14) angeordnet ist und mit einer zusammenpassenden Verbindungseingriffsstruktur an dem Adapter (24) in Eingriff bringbar ist, wobei die Strukturen jeweilige Antriebs- und Verbindungseingriffsflächen (48, 46, 56, 54) haben, wobei die Antriebsflächen in Antwort auf eine axiale Bewegung des Kolbens (14) auf das distale Ende des Gehäuses (12) zu in Eingriff bringbar sind und bei Eingriff gemeinsam bewegbar sind, um in Antwort auf eine relative Drehbewegung des Kolbens (14) und des Gehäuses (12) eine Drehbewegung des Adapters (24) relativ zu dem Gehäuse (12) zu ermöglichen, um zu bewirken, daß der Adapter (24) sich von dem distalen Ende des Gehäuses (12) trennt, und wobei die Verbindungsflächen (48, 56) miteinander in Eingriff bringbar sind, um den Kolben und den Adapter miteinander zu verbinden und den Adapter (24) zu befähigen, wenn er in Antwort auf eine gemeinsame Drehbewegung des Adapters (24) und des Kolbens (14) relativ zu dem Gehäuse (12) von dem Ende des Gehäuses (12) getrennt ist, in Reaktion auf eine gemeinsame axiale Bewegung des Kolbens (14) und des Adapters (24) in eine Richtung von dem distalen Ende des Gehäuses (12) weg mit der Nadel in das Innere des Gehäuses zurückgezogen zu werden.

10. Spritze nach Anspruch 9, wobei die Adaptereingriffsstruktur am distalen Ende des Kolbens (14) und die zusammenpassende Verbindungseingriffsstruktur an dem Adapter (24) jeweilige Flächen aufweisen, die zusammenwirken können, um in Antwort auf eine axiale Bewegung des Kolbens (14) auf das distale Ende des Gehäuses zu eine relative Drehbewegung des Kolbens (14) und des Adapters (24) zu bewirken.

11. Spritze nach Anspruch 9 oder 10, wobei die Adaptereingriffsstruktur am distalen Ende des Kolbens (14) und die zusammenpassende Verbindungseingriffsstruktur an dem Adapter (24) jeweilige Ausrichtungsflächen (44, 52) aufweisen, die von den Antriebsflächen (46, 54) verschieden sind und zusammenwirken können, um in Antwort auf eine axiale Bewegung des Kolbens auf das distale Ende des Gehäuses zu eine relative Drehbewegung des Kolbens und des Adapters zu bewirken.

## Revendications

1. Seringue comprenant
un corps de pompe creux (12) de forme allongée;
un élément (18) porté par le corps pour le montage d'une aiguille à son extrémité distale et pour l'établissement d'une communication, pour le fluide, avec l'intérieur du corps creux (12);
et un piston (14) mobile dans le corps entre des positions enfoncée et de retrait;
caractérisée en ce que la seringue comprend aussi
un joint d'étanchéité (72,74) entra le piston et le corps, à l'extrémité proximale du corps (12), pour rendre étanche la cavité intérieure du corps (12) à son extrémité proximale lorsque le piston (14) est dans sa position enfoncée;
et un moyen (20) pour fermer hermétiquement le corps à son extrémité distale,
lesdits moyens d'étanchéité (72,74,20) étant tels qu'ils permettent que des conditions stériles soient maintenues dans la seringue avant son utilisation.

2. Seringue selon la revendication 1, dans laquelle ledit élément (18) est fixé a l'extrémité distale (26) du corps (12) et porte l'aiguille en saillie sur lui, et dans laquelle ledit moyen de fermeture hermétique à l'extrémité distale du corps est constitué par un protège-aiguille (20) qui engaine l'aiguille et est fixé de manière étanche audit corps.

3. Seringue selon la revendication 1 ou 2, dans laquelle ledit moyen de fermeture hermétique (20) à l'extrémité distale du corps est constitué par un capuchon vissé sur l'extrémité distale du corps.

4. Seringue selon la revendication 3, comprenant des moyens (308,310) raccordés au corps et au capuchon pour indiquer une tentative de déplacement du piston et du capuchon l'un par rapport à l'autre.

5. Seringue selon la revendication 2, dans laquelle il est prévu des moyens, raccordés au corps et au protège-aiguille, pour indiquer une tentative de déplacement du protège-aiguille et du corps l'un par rapport a l'autre.

6. Seringue selon l'une quelconque des revendications 1 à 5, comprenant des moyens (310), reliant le corps et le piston en position enfoncée de celui-ci, pour indiquer une tentative de déplacement du piston et du corps l'un par rapport a l'autre.

7. Seringue selon l'une quelconque des revendications 1 à 6, dans laquelle le joint d'étanchéité à l'extrémité proximale comprend une nervure (72) sur l'un dudit corps et dudit piston et, sur l'autre dudit corps et dudit piston, un moyen (74) qui coopère avec ladite nervure pour former ledit joint d'étanchéité.

8. Seringue selon l'une quelconque des revendications 1 à 7, dans laquelle l'extrémité proximale du piston a la forme d'un cylindre creux et comporte une structure d'assemblage dimensionnée et conçue de façon a coopérer avec une structure d'assemblage complémentaire prévue a l'extrémité distale du corps, et dans laquelle le piston comporte en outre, entre ses extrémités proximale et distale, une structure pour assembler de manière détachable lesdites extrémités proximale et distale, de telle sorte que, quand le piston est retiré de l'extrémité distale du corps, l'extrémité proximale du piston puisse être détachée du piston et raccordée à l'extrémité distale du corps.

9. Seringue selon l'une quelconque des revendications 1 à 8, comprenant un adaptateur (24) qui est porté par le corps (12) à proximité de son extrémité distale et qui petit en être détaché par rotation par rapport au corps (12), l'adaptateur portant une aiguille, et dans laquelle la seringue comporte une structure d'accouplement pour l'adaptateur, disposée à l'extrémité distale du piston (14) et pouvant être mise en prise avec une structure conjuguée d'accouplement et de liaison prévue sur l'adaptateur (24), lesdites structures comportant des surfaces respectives d'entraînement et de liaison (48,46,56,54), lesdites surfaces d'entraînement pouvant être mises en prise l'une avec l'autre par déplacement axial dudit piston (14) vers l'extrémité distale dudit corps (12) et pouvant être déplacées ensemble, lorsqu'elles sont mises en prise, pour permettre une rotation de l'adaptateur (24) par rapport au corps (12) en réponse à une rotation relative du piston (14) et du corps (12), afin d'amener l'adaptateur (24) à se séparer de l'extrémité distale du corps (12), et dans laquelle les surfaces de liaison (48,56) peuvent être mises en prise l'une avec l'autre pour relier le piston et l'adaptateur l'un à l'autre et permettre à l'adaptateur (24), lorsqu'il s'est séparé de l'extrémité du corps (12) en réponse à la rotation conjointe dudit adaptateur (24) et dudit piston (14) par rapport audit corps (12), d'être rentré avec l'aiguille à l'intérieur du corps en réponse à un déplacement axial conjoint dudit piston (14) et dudit adapateur (24) dans la direction de leur éloignement de l'extrémité distale du corps (12).

10. Seringue selon la revendication 9, dans laquelle la structure d'accouplement avec l'adaptateur, située à l'extrémité distale du piston (14), et la structure conjuguée d'accouplement et, de liaison prévue sur l'adaptateur (24) comprennent des surfaces respectives qui peuvent coopérer pour produire une rotation relative du piston (14) et de l'adaptateur (24) en réponse au déplacement axial du piston (14) vers l'extrémité distale du corps.

11. Seringue selon la revendication 9 ou 10, dans laquelle la structure d'accouplement avec l'adaptateur, située à l'extrémité distale du piston (14), et la structure conjuguée d'accouplement et de liaison prévue sur l'adaptateur (24) comprennent des surfaces respectives d'alignement (44,52) différentes des surfaces d'entraînement (46,54) et pouvant coopérer pour produire une rotation relative du piston et de l'adaptateur en réponse au déplacement axial du piston vers l'extrémité distale du corps.
